# EUROPEAN PATENT APPLICATION

(11) **EP 0 548 370 A1**
(43) Date of publication of application: **30.06.1993**
(21) Application number: 92914709.8
(22) Date of filing: 14.07.1992
(51) Int. Cl.: A61K 31/55, C07D 243/00

(54) **DRUG FOR PREVENTING AND/OR TREATING ALLERGIC DISEASE**

(30) Priority: 15.07.1991 JP 174007/91
(71) Applicant: MOCHIDA PHARMACEUTICAL CO., LTD., Shinjuku-ku Tokyo 160 (JP)
(72) Inventor: MOCHIZUKI, Hidenori, Mochida Pharmaceutical Co.,, Tokyo 160 (JP); ISHIKAWA, Hiroshi, Mochida Pharmaceutical Co.,, Tokyo 160 (JP)
(74) Representative: Joly, Jean-Jacques
(86) International application number: JP9200896
(87) International publication number: WO9301815

(57) **Abstract**

A drug selectively inhibiting the production of an IgE antibody and useful for preventing and/or treating various allergic diseases, which contains as the active ingredient a lowly toxic 1-phenyl-5H-2,3-benzodiasepine derivative, represented by general formula (I), which is efficacious in treating allergic deseases, wherein R¹, R², R⁵ and R⁶ are each independently selected among hydrogen, hydroxyl, methoxy and ethoxy; and R³ and R⁴ are each independently selected among hydrogen, methyl and ethyl.

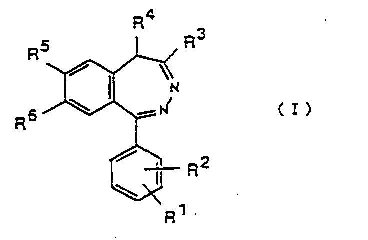

## Description

### TECHNICAL FIELD

This invention relates to a preventive and/or therapeutic drug for allergic diseases caused by immunoglobulin E antibodies (to be referred to as "IgE antibodies" hereinafter), such as certain types of bronchial asthma, conjunctivitis, rhinitis, dermatitis, hypersensitivity and the like.

### BACKGROUND ART

Allergic reactions in various allergic diseases such as certain types of bronchial asthma, conjunctivitis, rhinitis, dermatitis, hypersensitivity and the like occur as the results of the binding of certain antigens to corresponding IgE antibodies on the cellular surface of mast cells or basophils and subsequent release of various chemical mediators from these cells, such as prostaglandins, leukotrienes, thromboxanes, platelet activation factors and the like. Though symptomatic therapy is used most generally as therapeutic means for various symptoms which can be observed as the results of allergic reactions, causal treatment is also used commonly making use of compounds which inhibit releasing step of chemical mediators in a series of allergic reactions, such as sodium cromoglycate, tranilast, ketotifen, azelastin and the like. However, since little is known about a compound which can intercept a reaction step before the chemical mediator-releasing step, great concern has been directed toward the development of such a compound and its application to drugs. Among reaction steps in a series of allergic reactions, a compound which can inhibit especially the production step of IgE antibodies is considered to be useful as one of therapeutic drugs for the causal treatment.

It has been recognized that a compound represented by the following formula (I) is possessed of a function to improve, mainly centrally and partially peripherally, disturbance of autonomic nerve functions and functional and organic disturbances of the internal organs caused by a stimulation such as stress or the like, and 1-(3,4-dimethoxyphenyl)-5-ethyl-7,8-dimethoxy-4-methyl-5H-2,3-benzodiazepine (general name, tofisopam) as a typical example of the compounds has already been made into therapeutic drugs, based on its pharmacological actions as described above, for the treatment of autonomic nerve-related symptoms such as autonomic imbalance, head and neck injuries, climacteric disturbance, headache as an ovarian deficiency symptom, heavy feeling in the head, weariness, cardiopalmus, sweating and the like. 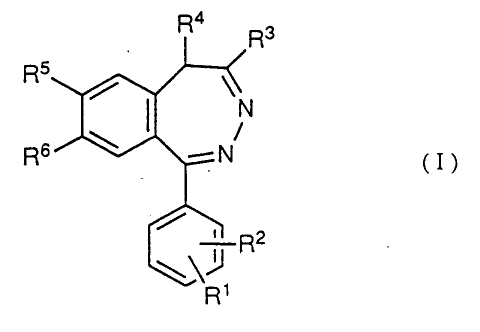
(In the above formula (I), R¹, R², R⁵ and R⁶ are independently selected from hydrogen atom, hydroxyl group, methoxy group and ethoxy group, and R³ and R⁴ are independently selected from hydrogen atom, methyl group and ethyl group.)

In addition, the following compounds are known as biological metabolites of tofisopam (*Iyakuhin Kenkyu*, vol.12, p.610, 1981).

1-(3-Methoxy-4-hydroxyphenyl)-5-ethyl-7,8-dimethoxy-4-methyl-5H-2,3-benzodiazepine, 1-(3,4-dimethoxyphenyl)-5-ethyl-7-hydroxy-8-methoxy-4-methyl-5H-2,3-benzodiazepine, 1-(3-methoxy-4-hydroxyphenyl)-5-ethyl-7-hydroxy-8-methoxy-4-methyl-5H-2,3-benzodiazepine, 1-(3,4-dihydroxyphenyl)-5-ethyl-7-hydroxy-8-methoxy-4-methyl-5H-2,3-benzodiazepine and 1-(3,4-dihydroxyphenyl)-5-ethyl-7,8-dihydroxy-4-methyl-5H-2,3-benzodiazepine.

Among reports so far published showing clinical applicability of the compound represented by the above formula (I), the following reports contain data on the effect of the compound in patients of allergic diseases.

According to a first report, only one case was improved when tofisopam was administered to 26 cases of patients likely having psychogenic bronchial asthma (*Iryo*, vol.44, p.385, 1990). This case, however, does not indicate the anti-allergic function of tofisopam, because this patient has been treated by simultaneous administration of a β₂ stimulant and a theophylline preparation as therapeutic drugs for bronchial asthma, and tests in this report have been made aiming at the central action of tofisopam and using patients of neurosis.

According to a second report, an efficacy of 50% was found in subjective symptoms and 25% in objective findings when tofisopam was administered to 16 cases of bronchial asthma patients (Y*akuri-to-Chiryo*, vol.19, p.2023, 1991). However, distinct autonomic abnormalities were found in the majority of the patients as the results of psychological tests, and many of the items used as efficiency markers were based on the autonomic abnormalities. In addition, this report describes that tests were carried out aiming at autonomic functional abnormalities as a cause of bronchial asthma and, as the results, a high efficiency was found in psychosomatic type patients. In consequence, this report deals with the efficiency of tofisopam on asthma patients as a case of psychosomatic disease, describes nothing about patients of asthma caused mainly by the increase in IgE and, similar to the first report, does not indicate the anti-allergic function of tofisopam.

According to a third report, 8 cases were improved when tofisopam was administered to 14 cases of chronic urticaria patients probably caused by psychosomatic disease (*Shinshin Iryo*, vol.2, p.1394, 1990). However, urticaria is roughly divided into allergic and non-allergic cases based on the onset mechanism, and the former case progresses as acute urticaria and the latter case frequently passes into chronic urticaria remaining the cause unknown. The disease of interest examined in this report, therefore, is non-allergic urticaria. In addition, the authors of this report have diagnosed these patients as autonomic imbalance, used tofisopam from a therapeutic viewpoint of autonomic imbalance and simultaneously administered an antihistaminic drug, a Chinese orthodox medicine and the like to each patient. In consequence, this report also does not indicate the efficiency of tofisopam on allergic diseases.

According to another report, 1 case was improved significantly when tofisopam was administered to 2 cases of chronic urticaria patients (*Rinsho-to-Kenkyu*, vol.65, p.1307, 1988). On the basis of the same reasons as the case of the aforementioned third report, this report also does not indicate the efficiency of tofisopam on allergic diseases. Thus, with regard to the compound represented by the aforementioned formula (I), there are no reports except for its usefulness as a therapeutic drug for the treatment of autonomic nerve-related symptoms, and virtually nothing is known about its effects on various allergic diseases which are believed to be related to IgE antibodies.

### DISCLOSURE OF THE INVENTION

It has been confirmed by animal experiments and clinical tests that production of IgE antibodies is induced by the sensitization of certain antigens and continues for a prolonged period of time in many cases. In consequence, an allergic disease-curing drug which can inhibit a step of a series of allergic reactions, especially the production step of IgE antibodies, is considered to be very useful. Such a therapeutic drug should have a function to inhibit not only the IgE antibody production at the stage of immune response induction but also prolonged IgE antibody production thereafter.

In addition, since not only IgE antibodies but also various other immunoglobulin classes take important roles in terms of host defense, a compound which selectively inhibits only IgE antibody production is preferable as a therapeutic drug for the treatment of allergic diseases.

A primary object of the present invention is to provide a therapeutic drug for the treatment of allergic diseases, which contains a compound as an active ingredient that hardly exerts influences upon the production of immunoglobulin classes taking important roles in host defense except for IgE antibodies, but can selectively inhibit prolonged production of IgE antibodies.

The inventors of the present invention have conducted studies on the screening of a compound which hardly exerts influences upon the production of immunoglobulin classes taking important roles in host defense except for IgE antibodies, but can selectively inhibit prolonged production of IgE antibodies. The present invention has been accomplished as a result of such efforts, based on the finding that a 1-phenyl-5H-2,3-benzodiazepine derivative represented by the following formula (I) was possessed of the aforementioned characteristics.

According to the present invention, there is provided a preventive and/or therapeutic means for allergic diseases which comprises using a 1-phenyl-5H-2,3-benzodiazepine derivative represented by the following formula (I) as an active ingredient. 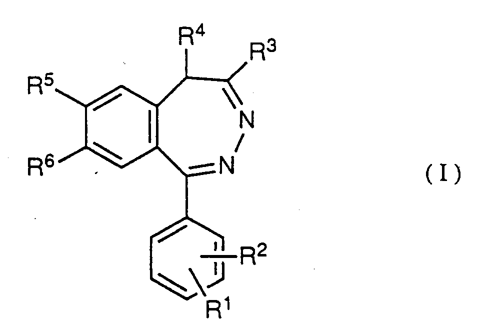
(In the above formula (I), R¹, R², R⁵ and R⁶ are independently selected from hydrogen atom, hydroxyl group, methoxy group and ethoxy group, and R³ and R⁴ are independently selected from hydrogen atom, methyl group and ethyl group.)

Another object of the present invention is to provide a process which comprises using a 1-phenyl-5H-2,3-benzodiazepine derivative represented by the above formula (I) as an active ingredient for the production of a preventive and/or therapeutic drug for allergic diseases.

Still another object of the present invention is to provide a process for the production of a preventive and/or therapeutic drug for allergic diseases which comprises preparing a composition useful for the prevention and/or therapy of allergic diseases by mixing a pharmacologically acceptable carrier with a 1-phenyl-5H-2,3-benzodiazepine derivative represented by the above formula (I) as an active ingredient that can be obtained by usually used means.

A further object of the present invention is to provide a preventive and/or therapeutic drug for allergic diseases which comprises a 1-phenyl-5H-2,3-benzsodiazepine derivative represented by the above formula (I) as an active ingredient.

Preferably, the 1-phenyl-5H-2,3-benzodiazepine derivative described above is 1-(3,4-dimethoxyphenyl)-5-ethyl-7,8-dimethoxy-4-methyl-5H-2,3-benzodiazepine.

### BEST MODE OF CARRYING OUT THE INVENTION

The following describes the present invention in detail.

The active ingredient to be used in the preventive and/or therapeutic drug of the present invention is selected from 1-phenyl-5H-2,3-benzodiazepine derivatives represented by the aforementioned formula (I). Since each of these benzodiazepine derivatives hardly exerts influences upon the production of immunoglobulin classes taking important roles in host defense except for IgE antibodies but can selectively inhibit prolonged production of IgE antibodies, it can be applied to the causal treatment of allergic diseases.

Though any of the 1-phenyl-5H-2,3-benzodiazepine derivatives represented by the aforementioned formula (I) is useful as the active ingredient of the allergic disease-treating drug of the present invention, 1-(3,4-dimethoxyphenyl)-5-ethyl-7,8-dimethoxy-4-methyl-5H-2,3-benzodiazepine may be most preferable. Also useful are the aforementioned biological metabolites.

Though not particularly limited, the 1-phenyl-5H-2,3-benzodiazepine derivative represented by the aforementioned formula (I) may be synthesized preferably in accordance with the process disclosed in Japanese Patent Application Kokai No. 55-92377.

In general, in addition to the 1-phenyl-5H-2,3-benzodiazepine derivative represented by the aforementioned formula (I), the therapeutic drug of the present invention for use in the treatment of allergic diseases may contain usually used appropriate carriers or media such as sterile water, plant oil and the like and physiologically acceptable solvents and solubilizing agents such as ethanol, glycerol, propylene glycol and the like, as well as a filler, a binder, a lubricant, a coloring agent, a flavor agent, an emulsifying or suspending agent (for example, Tween 80, acacia or the like) and other additives.

Examples of dosage forms include tablets, pills, capsules, granules, fine subtilaes, powders, suppositories, medicated syrups, lemonades, elixirs, ointments, solutions of eye-drops, aqueous or non-aqueous injections, emulsions or suspensions for injection and the like, as well as solid injections which are dissolved, emulsified or suspended prior to their use. Administration of the inventive drug may be effected either orally or parenterally (for example, by intravenous injection, intramuscular injection, subcutaneous injection, rectal administration, percutaneous absorption, transmucosal absorption or the like), but it may generally be administered orally to each patient.

The therapeutic drug of the present invention may be used in a dose sufficient enough to treat each allergic disease of interest, though the dose may vary depending on the dosage form, the route of administration and the number of administrations per day of the therapeutic drug, as well as the degree of symptoms, weight, age and the like of each patient. In general, it may be used in a dose of from 1 to 5000 mg/day, preferably from 50 to 1000 mg/day, as the amount of the active ingredient.

The therapeutic drug of the present invention can be used for the prevention of allergic diseases, interception of the onset of these diseases, prevention of bad-turning of symptoms thereof and improvement and treatment of the symptoms. In this instance, the allergic diseases to be treated with the therapeutic drug of the present invention are those caused by IgE antibodies, such as bronchial asthma, hay fever, angioneurotic edema, urticaria, serous otitis media, atopic dermatitis, pollinosis, allergic rhinitis, allergic enterogastritis, food allergy, drug allergy and the like.

In the therapeutic means of the present invention, a 1-phenyl-5H-2,3-benzodiazepine derivative represented by the aforementioned formula (I) is used as an active ingredient, and production of IgE antibodies at the stage of immune response induction and subsequent prolonged IgE antibody production are inhibited by allowing the active component to act as a selective inhibitor of these production steps.

In the process of the present invention, a 1-phenyl-5H-2,3-benzodiazepine derivative represented by the aforementioned formula (I) is used as an active ingredient for the production of a preventive and/or therapeutic drug for allergic diseases, and the derivative may be used alone or together with the aforementioned carrier or medium, if necessary by supplementing further with additive agents.

In the process for the production of the composition of the present invention, the composition is obtained by using a 1-phenyl-5H-2,3-benzodiazepine derivative represented by the aforementioned formula (I) alone or by uniformly mixing the derivative with the aforementioned carrier or medium, if necessary by supplementing further with additive agents.

### EXAMPLES

Efficacy of the allergic disease therapeutic drug of the present invention is explained illustratively based on the following test examples, using tofisopam as an example of the 1-phenyl-5H-2,3-benzodiazepine derivative represented by the aforementioned formula (I) which is the active ingredient of the therapeutic drug.

### (Test Example 1) Examination of the effect of tofisopam on the production of various types of immunoglobulin in mice

Effects of tofisopam on the production of various types of immunoglobulin were examined in the following manner.

That is, 4 mg of aluminum hydroxide to which 10 µg of ovalbumin has been adsorbed was intraperitoneally administered to 5 individuals per group of BALB/c line male mice (body weight, 21 - 25 g) to effect sensitization. Immediately after the sensitization, a 5% acacia aqueous solution containing a varied concentration of tofisopam or a 5% acacia aqueous solution containing no tofisopam (control) was administered orally once a day, 7 times in total.

After 14 days of the sensitization, blood samples were collected to measure produced amounts of various types of immunoglobulin by means of enzyme-linked immunosorbent assay (ELISA).

Production yield of IgE antibodies was measured in the following manner. That is, a blood plasma sample was added to immobilized anti-mouse IgE antibodies to allow IgE to be linked, followed by the reaction with biotin-labeled anti-mouse IgE antibodies and avidin-labeled horseradish peroxidase, and then the amount of produced IgE antibodies was measured based on the horseradish peroxidase activity as a marker (*Men'eki-to-Shikkan*, vol.15, pp.211 - 216, 1988). The results are shown in Table 1.

Production yields of IgG₁ and IgM antibodies were measured in the following manner. That is, a blood plasma sample was added to immobilized anti-mouse IgG₁ or IgM antibodies to allow IgG₁ antibodies or IgM antibodies to be linked, followed by the reaction with horseradish peroxidase-labeled anti-mouse IgG₁ antibodies or horseradish peroxidase-labeled anti-mouse IgM antibodies, and then the amount of produced IgG₁ or IgM antibodies was measured based on the horseradish peroxidase activity as a marker. The results are shown in Tables 2 and 3, respectively.

**Table 1**

| Drug tested | Dose (mg/kg/day) | IgE antibody yield (% control) |
|---|---|---|
| Control | - | 100 |
| Tofisopam | 100 | 66 |
| Tofisopam | 300 | 56 |

**Table 2**

| Drug tested | Dose (mg/kg/day | IgG₁ antibody yield (% control) |
|---|---|---|
| Control | - | 100 |
| Tofisopam | 100 | 113 |
| Tofisopam | 300 | 111 |

**Table 3**

| Drug tested | Dose (mg/kg/day) | IgM antibody yield (% control) |
|---|---|---|
| Control | - | 100 |
| Tofisopam | 100 | 97 |
| Tofisopam | 300 | 87 |

It is evident from these results that, when tofisopam is orally administered to ovalbumin-sensitized mice of BALB/c line for 7 days with a dose of 100 or 300 mg/kg/day, it inhibits approximately 34 or 44% of the IgE antibody production, while it hardly exerts influence upon the production of IgG₁ and IgM antibodies.

### (Test Example 2) Examination of the effect of tofisopam on rat experimental asthma

Effects of tofisopam on rat experimental asthma were examined in the following manner.

That is, 2 x 10¹⁰ cells of inactivated *Bordetella pertussis* and 1 mg of ovalbumin were respectively administered intraperitoneally and intramuscularly to 5 individuals per group of Wister line male rats (body weight, 194 - 210 g) to effect sensitization. Immediately after the sensitization, a 5% acacia aqueous solution containing a varied concentration of tofisopam or a 5% acacia aqueous solution containing no tofisopam (control) was administered orally once a day, 14 times in total.

After 14 days of the sensitization, ovalbumin was administered to the sensitized rats intravenously at a dose of 0.5 mg/kg, and the increasing degree of airway resistance was measured by means of the over flow technique (*Arch. Exp*. *Pathol. Pharmak.*, vol.195, pp.71 - 74, 1974). The thus obtained inhibition rate of airway contraction, when the result of the control test is defined as 100, is shown in Table 4.

**Table 4**

| Drug tested | Dose (mg/kg/day) | Inhibition rate (%) |
|---|---|---|
| Tofisopam | 100 | 27 |

On the basis of this result, it was confirmed that tofisopam can inhibit the airway reaction when it is orally administered to ovalbumin-sensitized male rats of Wister line for 14 days with a dose of 100 mg/kg/day.

As was evident from the above test results, the 1-phenyl-5H-2,3-benzodiazepine derivative represented by the aforementioned formula (I) which was the active ingredient of the allergic disease-curing drug of the present invention inhibits IgE antibody production in mice, but hardly exerted influence upon the production of IgG₁ and IgM antibodies. In rats, the derivative showed anti-asthma function.

Test results on the acute, subacute and chronic toxicities of tofisopam as a typical example of the 1-phenyl-5H-2,3-benzodiazepine derivative represented by the aforementioned formula (I) have already been reported, which confirmed safety of the compound *(Iyakuhin Kenkyu*, vol.12, p.547, 1981).

In consequence, it is evident that the allergic disease preventive and/or therapeutic drug of the present invention which comprises a 1-phenyl-5H-2,3-benzodiazepine derivative represented by the above formula (I) as an active ingredient can be used as a drug for the treatment and prevention of allergic diseases caused by IgE antibodies, such as certain types of bronchial asthma, conjunctivitis, rhinitis, dermatitis, hypersensitivity and the like.

### INDUSTRIAL APPLICABILITY

According to the present invention, there is provided a preventive and/or therapeutic means for allergic diseases, which comprises using a specified benzodiazepine derivative as an active ingredient that hardly exerts influences upon the production of immunoglobulin classes taking important roles in host defense except for IgE antibodies, but can selectively inhibit prolonged production of IgE antibodies, as well as a process in which this derivative is used as an active ingredient, a preventive and/or therapeutic drug which contains the derivative as an active ingredient and a process for the production thereof.

Since the present invention uses a 1-phenyl-5H-2,3-benzodiazepine derivative represented by the aforementioned formula (I), and the derivative can inhibit production of IgE antibodies selectively as described above, the therapeutic drug of the present invention for the treatment of allergic diseases shows effective activities against various allergic diseases as a causal treatment drug and can be used for the prevention of allergic diseases, interception of the onset of these diseases, prevention of bad-turning of symptoms thereof and improvement and treatment of the symptoms.

## Claims

1. A preventive and/or therapeutic method for allergic diseases which comprises using a 1-phenyl-5H-2,3-benzodiazepine derivative represented by the following formula (I) as an active ingredient: 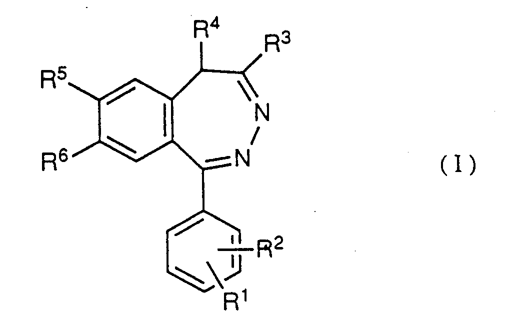 wherein R¹, R², R⁵ and R⁶ are independently selected from hydrogen atom, hydroxyl group, methoxy group and ethoxy group, and R³ and R⁴ are independently selected from hydrogen atom, methyl group and ethyl group.

2. The preventive and/or therapeutic method for allergic diseases according to claim 1 wherein said 1-phenyl-5H-2,3-benzodiazepine derivative is 1-(3,4-dimethoxyphenyl)-5-ethyl-7,8-dimethoxy-4-methyl-5H-2,3-benzodiazepine.

3. A process in which a 1-phenyl-5H-2,3-benzodiazepine derivative represented by the following formula (I) is used as an active ingredient for the production of a preventive and/or therapeutic drug for allergic diseases: 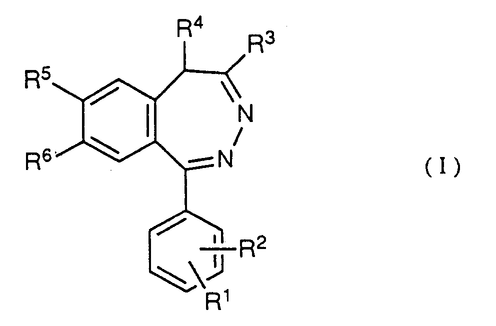 wherein R¹, R², R⁵ and R⁶ are independently selected from hydrogen atom, hydroxyl group, methoxy group and ethoxy group, and R³ and R⁴ are independently selected from hydrogen atom, methyl group and ethyl group.

4. The process according to claim 3 wherein said 1-phenyl-5H-2,3-benzodiazepine derivative is 1-(3,4-dimethoxyphenyl)-5-ethyl-7,8-dimethoxy-4-methyl-5H-2,3-benzodiazepine.

5. A process for producing a preventive and/or therapeutic drug for allergic diseases which comprises preparing a composition useful for the prevention and/or therapy of allergic diseases by mixing a pharmacologically acceptable carrier with a 1-phenyl-5H-2,3-benzodiazepine derivative represented by the following formula (I) as an active ingredient that can be obtained by usually used methods: 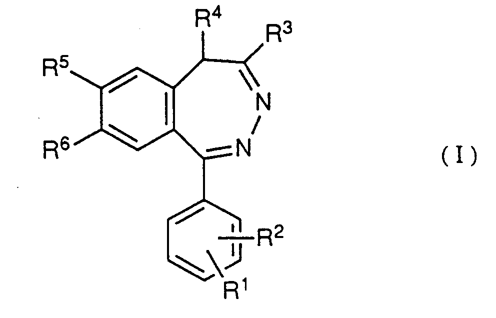 wherein R¹, R², R⁵ and R⁶ are independently selected from hydrogen atom, hydroxyl group, methoxy group and ethoxy group, and R³ and R⁴ are independently selected from hydrogen atom, methyl group and ethyl group.

6. The production process according to claim 5 wherein said 1-phenyl-5H-2,3-benzodiazepine derivative is 1-(3,4-dimethoxyphenyl)-5-ethyl-7,8-dimethoxy-4-methyl-5H-2,3-benzodiazepine.

7. A preventive and/or therapeutic drug for allergic diseases which comprises a 1-phenyl-5H-2,3-benzodiazepine derivative represented by the following formula (I) as an active ingredient: 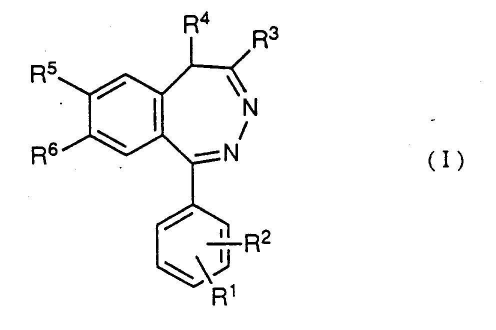 wherein R¹, R², R⁵ and R⁶ are independently selected from hydrogen atom, hydroxyl group, methoxy group and ethoxy group, and R³ and R⁴ are independently selected from hydrogen atom, methyl group and ethyl group.

8. The preventive and/or therapeutic drug for allergic diseases according to claim 7 wherein said 1-phenyl-5H-2,3-benzodiazepine derivative is 1-(3,4-dimethoxyphenyl)-5-ethyl-7,8-dimethoxy-4-methyl-5H-2,3-benzodiazepine.
